# EUROPEAN PATENT APPLICATION

(11) **EP 3 974 445 A1**
(43) Date of publication of application: **30.03.2022**
(21) Application number: 20809148.8
(22) Date of filing: 19.05.2020
(51) Int. Cl.: C07K 16/00, A61K 39/395, C07K 16/28, C12N 15/13, C12N 15/63

(54) **BISPECIFIC MOLECULE, AND PREPARATION AND USE THEREOF**

(30) Priority: 20.05.2019 CN 201910419818
(71) Applicant: Nantong Yichen Biopharma. Co. Ltd., Nantong, Jiangsu 226001 (CN)
(72) Inventor: WANG, Feng, Shanghai 200233 (CN); ZHENG, Huayang, Shanghai 200233 (CN); ZHANG, Yuhan, Shanghai 200233 (CN)
(74) Representative: Biggi, Cristina
(86) International application number: PCT/CN2020/091060
(87) International publication number: WO 2020/233571

(57) **Abstract**

Provided are a bispecific molecule and preparation and use thereof. The bispecific molecule includes a molecule that specifically binds an interleukin-1 receptor (IL-1R) and an antibody that targets a free inflammatory factor. The molecule that specifically binds the cell surface interleukin-1 receptor (IL-1R) aggregates the antibody that targets the free inflammatory factor linked thereto on or near the cell surface, thereby the local concentration of the bispecific molecule on or near the cell surface is increased, adverse reactions are avoided, treatment effectiveness is increased and the infection risk of patients is also reduced.

## Description

### Technical Field

The invention belongs to the field of biopharmaceuticals, and specifically relates to a bispecific molecule that specifically binds interleukin-1 receptor (IL-1R) and free inflammatory factors, and its preparation and use thereof.

### Background

Pro-inflammatory cytokines regulate various physiological processes, and their abnormal up-regulation thereof is the main cause of various inflammatory diseases. The use of recombinant anti-inflammatory cytokines, recombinant soluble receptors or neutralizing antibodies targeting inflammatory cytokines to treat the inflammatory diseases has achieved good clinical results already.

TNF-α is a pro-inflammatory cytokine produced endogenously in the early stage of trauma or bacterial infection. TNFR signaling pathway transduction is closely related to various inflammatory diseases such as rheumatoid arthritis (RA), Crohn's disease, atherosclerosis, psoriasis, sepsis, diabetes, obesity, eta. Etanercept is the first TNF-α inhibitor approved for the treatment of rheumatoid arthritis. Infliximab, which targets TNF-α, was also approved by the FDA in 1998 for the treatment of Crohn's disease. Infliximab binds monomeric (non-activated state) or trimerized (activated state) TNF-α, while Etanercept mainly binds activated TNF-α trimer. The fully human Adalimumab was approved by the FDA in 2002 for the treatment of rheumatoid arthritis. TNF-α is the main factor against intracellular bacterial infections. Therefore, while the above-mentioned TNF-α inhibitors show good clinical effects, they also inevitably increase risks of various infections such as tuberculosis, reactivation of HBV, herpes zoster virus (HZV) and the like. As immunosuppressive drugs, TNF-α inhibitors not only show good clinical effects, but also greatly increases the probability of patients with malignant hematological tumors.

Similar to the TNF-α family, the imbalance between an IL-1 family's pro-inflammatory signals and inhibiting inflammatory signals is also closely related to the occurrence of various chronic diseases, such as inflammatory bowel disease, rheumatoid arthritis, and auto-inflammatory diseases related to IL-1β overexpression. At present, 3 drugs targeting the IL-1 pathway (Anakinra, Canakinumab and Rilonacept) have already been approved by the FDA for marketing. Similar to Anakinra, Canakinumab and Rilonacept can effectively reduce the symptoms of inflammatory diseases. In addition, Canakinumab also shows good efficacy in the treatment of systemic juvenile idiopathic arthritis (SJIA). It is discovered from a meta-analysis of 4 clinical trials for the treatment of rheumatoid arthritis that the incidence of pneumonia, osteomyelitis, cellulitis, bursitis, herpes zoster, bunion, or gangrene increased significantly in Anakinra-treated patients. Therefore, the above-mentioned drugs for the IL-1 pathway are not recommended for patients with high infection risks.

IL-6 is another pluripotent pro-inflammatory factor in the immune system. It is secreted by a variety of cells and may stimulate B cells, NK cells, osteoclasts, and tumor cells. Therefore, it plays an important role in autoimmune diseases such as rheumatoid arthritis. Tocilizumab, a humanized antibody targeting IL-6, was approved for the treatment of RA and SJIA in 2010 and 2011, respectively. Siltuximab targeting IL-6R was approved by the FDA for human immunodeficiency virus (HIV) negative and human herpes virus (HHV-8) negative polycentric Castleman disease. However, clinical applications of these two targeted drugs also showed adverse effects, similar as TNF-α and IL-1 targeted drugs, such as greatly increased the probability of infection in patients. As a solution, the antibodies of Ustekinumab, Secukinumab, and ixekizumab targeting effector Th17 cytokines are required to receive preventively before treatment so as to avoid the risk of reactivation of tuberculosis (Rider P, Carmi Y and Cohen I. Iht J Cell Biol. 2016: 9259646).

To develop a bispecific antibody that simultaneously targets two or more of the above pathways, major pharmaceutical companies have made various attempts. Multiple bispecific antibodies that target TNF-α and IL-17A, such as ABT-12, COVA-322, LY3114062, and BCD-121 does not meet expectation during the clinical trials (Baker, KF. et al. Ann Rheum Dis. 2018, 77(2): 175-187). The latest phase-II clinical results of ABT-981 (Abbott company) targeting IL-1α and IL-1β showed that ABT-981 has no improvement on pain or imaging endpoints of erosive hand osteoarthritis (HOA), and has limited effect on pain relief of Western Ontario and McMaster Universities Osteoarthritis (WOMAC), no improvement effect on synovitis (Kloppengurg, M. et al. Ann Rheum Dis. 2019, 78: 413-420 and Fleischmann, RM. et al. Arthritis Rheumatol. 2019, doi: 10.1002/art.40840).

Therefore, there is an urgent need in the art for a bispecific antibody that can greatly reduce its adverse effects to systemic circulation, while improving its clinical efficacy in treating the inflammatory diseases.

### Summary

The present invention provides a bispecific molecule and preparation and use thereof.

At present, most drugs targeting pro-inflammatory factors are aimed at free inflammatory factors in the body. These drugs bind inflammatory factors and circulate throughout the body. Therefore, they may cause systemic adverse effects and increase the risk of infection. It is discovered by the inventor that with a molecule that specifically binds to cell surface inflammatory factor receptor as a mediator, an antibody targeting the free inflammatory factor can be confined to or near the cell surface, exerting synergistic and local anti-inflammatory effects of the two moieties and effectively avoiding possible adverse effects of bispecific molecules in the systemic circulation.

Based on the above findings, the present invention provides a bispecific molecule, wherein the bispecific molecule includes a molecule that specifically binds IL-1R and an antibody that targets a free inflammatory factor, wherein the molecule that specifically binds IL-1R and the antibody that targets the free inflammatory factor are connected by a linker peptide. The bispecific molecule of the present invention is enriched by the molecule that specifically binds to IL-1R, and its circulation through the body are greatly prevented. The antibody targeting the free inflammatory factor may further neutralize the free inflammatory factor that are locally increased due to inflammation on or near the surface of the cells expressing IL-1R, synergistically enhancing the anti-inflammatory response of the molecule that specifically binds IL-1R, thus avoiding the adverse reactions produced by the antibody targeting the free inflammatory factor.

In some embodiments, the molecule that specifically binds IL-1R is a non-immunoglobulin polypeptide. In some embodiments, the non-immunoglobulin polypeptide that specifically binds IL-1R is IL-1RA. In some embodiments, the IL-1RA that specifically binds IL-1R has an amino acid sequence as shown in SEQ ID NO: 49 and/or an amino acid sequence as shown in SEQ ID NO: 50. In some embodiments, the non-immunoglobulin polypeptide that specifically binds IL-1R consists of an amino acid sequence having at least 85%-99% of identity with SEQ ID NO: 50.

In some embodiments, the molecule that specifically binds IL-1R is an anti-IL-1R antibody. In some embodiments, the anti-IL-1R antibody is a single domain antibody, a chimeric antibody, a humanized antibody, a human antibody, or a modified moiety of the above antibodies. In some embodiments, the anti-IL-1R antibody includes CDR groups: HCDR1, HCDR2, HCDR3, LCDR1, LCDR2 and LCDR3, wherein, HCDR1, HCDR2 and HCDR3 have the amino acid sequences as shown in SEQ ID NO: 52, SEQ ID NO: 56 or SEQ ID NO: 58; LCDR1, LCDR2 and LCDR3 have the amino acid sequences as shown in SEQ ID NO: 54 or SEQ ID NO: 60. In some embodiments, the antibody that specifically binds L-1R is consisting of the following heavy chains and light chains: a heavy chain shown in SEQ ID NO: 52 and a light chain shown in SEQ ID NO: 54; a heavy chain shown in SEQ ID NO: 56 and a light chain shown in SEQ ID NO: 54; or a heavy chain shown in SEQ ID NO: 58 and a light chain shown in SEQ ID NO: 60.

In some embodiments, the free inflammatory factor targeted by the bispecific molecule is selected from one of the following (not limited to): IL-1 superfamily (IL-Ia, IL-1β, IL-18, IL-33, IL-36a, IL-36β, IL-36γ), IL-4, IL-13, IL-17A, IL-17E, IL-6, IL-12, IL-23, TNF superfamily (TNFa, TNFβ, TNFγ, OX40L (TNFSF4), CD40L (CD154), FasL (CD178, CD95L), CD27L (CD70), CD30L(CD153), 4-1 BBL, CD253 (APO-2L), CD254, APO-3L(DR3L), CD256(TALL-2), CD257(B1yS), LIGHT(CD258), TL1 (TNFSF18, AITRL), ED1-A1), BAFF, IFN or GM-CSF.

In some embodiments, the antibody that targets the above free inflammatory factor is a chimeric antibody, a humanized antibody, a human antibody, or a modified fragment of the above antibodies.

In some specific embodiments, the free inflammatory factor which the bispecific molecule targets is IL-1β. In some embodiments, the antibody that targets the free inflammatory factor IL-1β has the HCDR1, HCDR2 and HCDR3 sequences contained in a heavy chain amino acid sequence shown in SEQ ID NO: 2, and the LCDR1, LCDR2 and LCDR3 sequences contained in a light chain amino acid sequence shown in SEQ ID NO: 4; or the HCDRI, HCDR2 and HCDR3 sequences contained in a heavy chain amino acid sequence shown in SEQ ID NO: 6, and the LCDR1, LCDR2 and LCDR3 sequences contained in a light chain amino acid sequence shown in SEQ ID NO: 8.

In some specific embodiments, the free inflammatory factor targeted by the bispecific molecule is IL-17A. In some embodiments, the antibody that targets the free inflammatory factor IL-17 has the HCDR1, HCDR2 and HCDR3 sequences contained in a heavy chain amino acid sequence shown in SEQ ID NO: 26, and the LCDR1, LCDR2 and LCDR3 sequences contained in a light chain amino acid sequence shown in SEQ ID NO: 28.

In some specific embodiments, the free inflammatory factor targets by the bispecific molecule are IL-6. In some embodiments, the antibody that targets the free inflammatory factor IL-6 has the HCDR1, HCDR2 and HCDR3 sequences contained in a heavy chain amino acid sequence shown in SEQ ID NO: 38, and the LCDR1, LCDR2 and LCDR3 sequences contained in a light chain amino acid sequence shown in SEQ ID NO:40.

In some embodiments, the bispecific molecule of the present invention includes a molecule that specifically binds IL-1R and an antibody that targets a free inflammatory factor IL-1β, and the molecule that specifically binds IL-1R and the antibody that targets the free inflammatory factor IL-1β are connected by a linker peptide. In some embodiments, the humanized antibody that targets the free inflammatory factor IL-1β has a heavy chain shown in SEQ ID NO: 2 and a light chain shown in SEQ ID NO: 4. In some embodiments, the humanized antibody that targets the free inflammatory factor IL-1β has a heavy chain as shown in SEQ ID NO: 6 and a light chain as shown in SEQ ID NO: 8. In embodiments, the molecule that specifically binds IL-1R is a non-immunoglobulin polypeptide. In some embodiments, the non-immunoglobulin polypeptide of the molecule that specifically binds IL-1R is IL-1RA. In some embodiments, the non-immunoglobulin polypeptide that specifically binds IL-1R has a nucleotide sequence shown in SEQ ID NO: 49 and/or has an amino acid sequence shown in SEQ ID NO: 50. In some embodiments, the non-immunoglobulin polypeptide that specifically binds IL-1R is consisting of an amino acid sequence having at least 85%-99% of identity with SEQ ID NO: 50. In some embodiments, the molecule that specifically binds IL-1R is an anti-IL-1R antibody. In some embodiments, the antibody that specifically binds IL-1R has a CDR group including: HCDR1, HCDR2, HCDR3, LCDR1, LCDR2, and LCDR3, wherein HCDR1, HCDR2, and HCDR3 have the amino acid sequence shown in SEQ ID NO: 52, SEQ ID NO: 56 or SEQ ID NO: 58; LCDR1, LCDR2, and LCDR3 have the amino acid sequence shown in SEQ ID NO: 54 or SEQ ID NO: 60. In some embodiments, the bispecific molecule that specifically binds IL-1R and IL-1β has a heavy chain and a light chain with the following amino acid sequences: SEQ ID NO: 10 and SEQ ID NO: 4; SEQ ID NO : 2 and SEQ ID NO: 12; SEQ ID NO: 14 and SEQ ID NO: 4; SEQ ID NO: 2 and SEQ ID NO: 16; SEQ ID NO: 18 and SEQ ID NO: 4; SEQ ID NO: 2 and SEQ ID NO: 20; SEQ ID NO: 6 and SEQ ID NO: 22; SEQ ID NO: 6 and SEQ ID NO: 24; SEQ ID NO: 62 and SEQ ID NO: 4; SEQ ID NO: 64 and SEQ ID NO: 4; SEQ ID NO: 66 and SEQ ID NO: 8; SEQ ID NO: 6 and SEQ ID NO: 68; SEQ ID NO: 70 and SEQ ID NO: 4; SEQ ID NO: 72 and SEQ ID NO: 4; SEQ ID NO: 74 and SEQ ID NO: 4; SEQ ID NO: 76 and SEQ ID NO: 4.

In some embodiments, the bispecific molecule of the present invention includes a molecule that specifically binds IL-1R and an antibody that targets a free inflammatory factor IL-17, and the molecule that specifically binds IL-1R and the antibody that targets the free inflammatory factor IL-17 are connected by a linker peptide. In some embodiments, the humanized antibody that targets the free inflammatory factor IL-17 has a heavy chain shown in SEQ ID NO: 26 and a light chain shown in SEQ ID NO: 28. In some embodiments, the molecule that specifically binds IL-1R is a non-immunoglobulin polypeptide. In embodiments, the non-immunoglobulin polypeptide that specifically binds IL-1R is IL-1RA. In some embodiments, the non-immunoglobulin polypeptide IL-1RA that specifically binds IL-1R has a nucleotide sequence shown in SEQ ID NO: 49 and/or has an amino acid sequence shown in SEQ ID NO: 50. In some embodiments, the non-immunoglobulin polypeptide that specifically binds IL-1R is consisting of an amino acid sequence having at least 85%-99% of identity with SEQ ID NO: 50. In some embodiments, the molecule that specifically binds IL-1R is an anti-IL-1R antibody. In some embodiments, the antibody that specifically binds IL-1R has a CDR group including: HCDR1, HCDR2, HCDR3, LCDR1, LCDR2, and LCDR3, wherein HCDR1, HCDR2, and HCDR3 have the amino acid sequence shown in SEQ ID NO: 52, SEQ ID NO: 56 or SEQ ID NO: 58; LCDR1, LCDR2, and LCDR3 have the amino acid sequence shown in SEQ ID NO: 54 or SEQ ID NO: 60. In some embodiments, the bispecific molecule that specifically binds IL-1R and IL-17 has a heavy chain and a light chain with the following amino acid sequences: SEQ ID NO: 34 and SEQ ID NO: 28; SEQ ID NO: 26 and SEQ ID NO: 36; SEQ ID NO: 30 and SEQ ID NO: 28; SEQ ID NO: 26 and SEQ ID NO: 32.

In some embodiments, the bispecific molecule of the present invention includes a molecule that specifically binds IL-1R and an antibody that targets a free inflammatory factor IL-6, and the molecule that specifically binds IL-1R and the antibody that targets the free inflammatory factor IL-6 are connected by a linker peptide. In some embodiments, the humanized antibody that targets the free inflammatory factor IL-6 has a heavy chain shown in SEQ ID NO: 38 and a light chain shown in SEQ ID NO: 40. In some embodiments, the molecule that specifically binds to IL-1R is a non-immunoglobulin polypeptide. In some embodiments, the non-immunoglobulin polypeptide of the molecule that specifically binds IL-1R is IL-1RA. In some embodiments, the non-immunoglobulin polypeptide IL-1RA that specifically binds to IL-1R has a nucleotide sequence shown in SEQ ID NO: 49 and/or has an amino acid sequence shown in SEQ ID NO: 50. In some embodiments, the non-immunoglobulin polypeptide that specifically binds IL-1R is consisting of an amino acid sequence having at least 85%-99% of identity with SEQ ID NO: 50. In some embodiments, the molecule that specifically binds IL-1R is an anti-IL-1R antibody. In some embodiments, the antibody that specifically binds to IL-1R has a CDR group including: HCDR1, HCDR2, HCDR3, LCDR1, LCDR2, and LCDR3, wherein HCDR1, HCDR2, and HCDR3 have the amino acid sequence shown in SEQ ID NO: 52, SEQ ID NO: 56 or SEQ ID NO: 58; LCDR1, LCDR2, and LCDR3 have the amino acid sequence shown in SEQ ID NO: 54 or SEQ ID NO: 60. In some embodiments, the bispecific molecule that specifically binds IL-1R and IL-6 has a heavy chain and a light chain with the following amino acid sequences: SEQ ID NO: 46 and SEQ ID NO: 40; SEQ ID NO: 38 and SEQ ID NO: 48; SEQ ID NO: 38 and SEQ ID NO: 44; SEQ ID NO: 42 and SEQ ID NO: 40.

In one aspect, the present invention provides a polynucleotide encoding the above bispecific molecule.

In some embodiments, the bispecific molecule that specifically binds IL-1R and targets the free inflammatory factor IL-1 β has a heavy chain and a light chain with the following nucleotide sequences: SEQ ID NO: 9 and SEQ ID NO : 3; SEQ ID NO: 1 and SEQ ID NO: 11; SEQ ID NO: 13 and SEQ ID NO: 3; SEQ ID NO: 1 and SEQ ID NO: 15; SEQ ID NO: 17 and SEQ ID NO: 3; SEQ ID NO: 1 and SEQ ID NO: 19; SEQ ID NO: 5 and SEQ ID NO: 21; SEQ ID NO: 5 and SEQ ID NO: 23; SEQ ID NO: 61 and SEQ ID NO: 3; SEQ ID NO: 63 and SEQ ID NO: 3; SEQ ID NO: 65 and SEQ ID NO: 7; SEQ ID NO: 5 and SEQ ID NO: 67; SEQ ID NO: 69 and SEQ ID NO: 3; SEQ ID NO : 71 and SEQ ID NO: 3; SEQ ID NO: 73 and SEQ ID NO: 3; SEQ ID NO: 75 and SEQ ID NO: 3..

In some embodiments, the bispecific molecule that specifically binds IL-1R and targets the free inflammatory factor IL-17 has a heavy chain and a light chain with the following nucleotide sequences: SEQ ID NO: 33 and SEQ ID NO: 27; SEQ ID NO: 25 and SEQ ID NO: 35; SEQ ID NO: 29 and SEQ ID NO: 27; SEQ ID NO: 25 and SEQ ID NO: 31.

In some embodiments, the bispecific molecule that specifically binds IL-1R and targets the free inflammatory factor IL-6 has a heavy chain and a light chain with the following nucleotide sequences: SEQ ID NO: 45 and SEQ ID NO: 39; SEQ ID NO: 37 and SEQ ID NO: 47; SEQ ID NO: 37 and SEQ ID NO: 43; SEQ ID NO: 41 and SEQ ID NO: 39.

In one aspect, the present invention provides an expression vector comprising the polynucleotide of the present invention.

In one aspect, the present invention provides a host cell comprising the expression vector of the present invention.

In one aspect, the present invention provides a pharmaceutical composition comprising the bispecific molecule of the present invention and a pharmaceutically acceptable carrier or formulation.

In another aspect, the present invention further provides a method for treating inflammatory diseases and/or autoimmune diseases in a subject in need thereof, the method includes administering to a subject a therapeutically effective amount of a composition, and the composition includes the bispecific molecule of the present invention in a pharmaceutically acceptable form.

As mentioned above, the present invention provides a bispecific molecule and its preparation and use. The antibody targeting free inflammatory factors (such as β, IL-17 or IL-6) was gathered on or near IL-1R-expressing cell surface by the molecule that specifically binds IL-1R (such as non-immunoglobulin polypeptide IL-1RA, and IL-1R antibody). so that the concentration of the bispecific molecules on or near the cell surface is locally increased. Benefited from high local concentration of bispecific targeting molecule, not only avoiding the adverse reactions caused by the high level systemic circulation of antibodies targeting one or more free inflammatory factors, but also improving the therapeutic index limited by single antigen targeting antibodies focusing on a unique inflammatory factor. Thus, the bispecific molecule of the present invention could greatly reduce the risk of patient infection while improve the effectiveness of treatment.

### Brief Description of the Drawings

Drawings constituting the present application are used to provide further understanding of the present invention, exemplary embodiments of the present invention and descriptions thereof are used to explain the present invention and do not constitute improper limitation to the present invention. In the drawings:

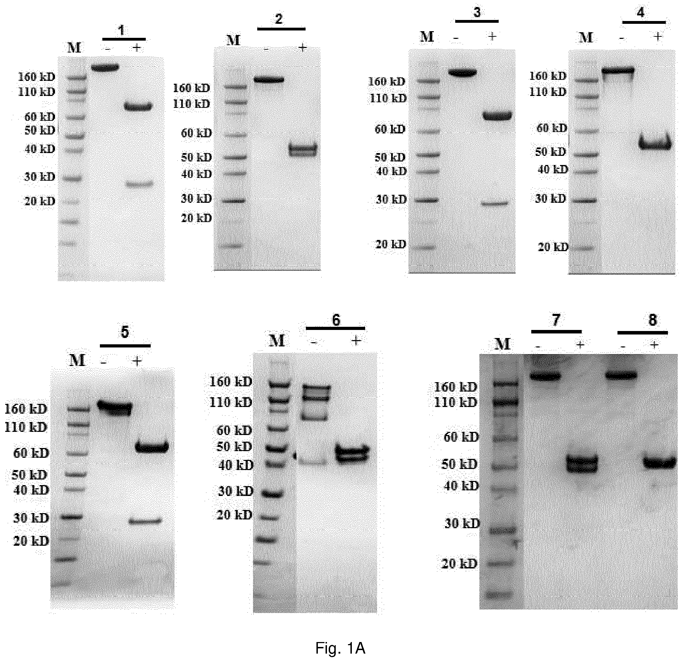

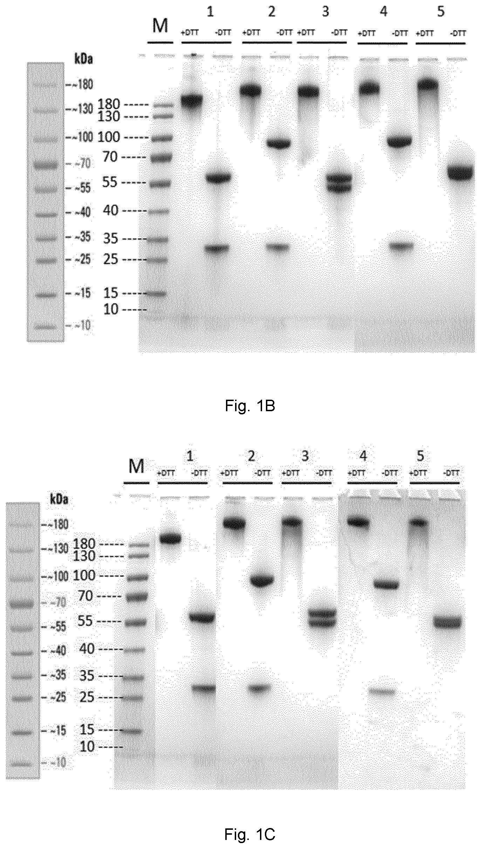

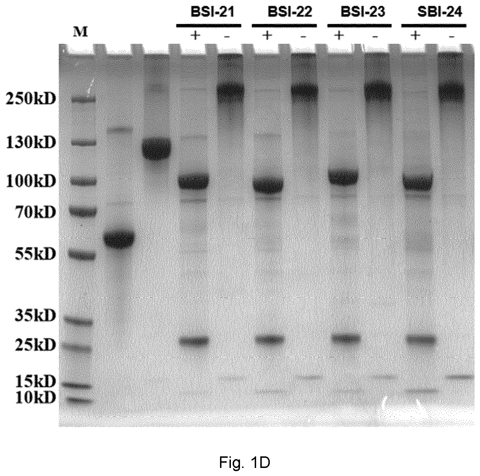

Fig. 1 is an SDS-PAGE diagram, herein M is a protein marker, "-" means that a reducing agent DTT is not added while a sample is loaded, and "+" means that the reducing agent DTT is added while the sample is loaded. Fig. 1A is an SDS-PAGE diagram of a bispecific molecule that specifically binds IL-1R and IL-1β, wherein Lanes 1-8 are: BSI-1, BSI-2, BSI-3, BSI-4, BSI-5, BSI-6, BSI-7, and BSI-8 respectively; Fig. 1B is an SDS-PAGE diagram of a bispecific molecule that specifically binds IL-1R and IL-17A, wherein Lane 1 is aIL-17AH, and Lanes 2-5 are: BSI -9, BSI-12, BSI-11, and BSI-10 respectively; Fig. 1C is an SDS-PAGE diagram of a bispecific molecule that specifically binds IL-1R and IL-6, wherein Lane 1 is aIL-6, Lanes 2-5 are: BSI-13, BSI-15, BSI-16, and BSI-14 respectively.
Fig. 2 is results of gel exclusion chromatography, wherein Fig. 2A is results of the gel exclusion chromatography of the bispecific molecule that specifically binds IL-1R and IL-17, Fig. 2B is results of the gel exclusion chromatography of the bispecific molecule that specifically binds IL-1R and IL-6.
Fig. 3 is ELISA results of the bispecific molecule that binds to recombinant human IL-1β, recombinant mouse IL-1β, recombinant human IL-1R, or recombinant mouse IL-1R.
Fig. 4 shows the inhibition of IL-1β-induced HEK-Blue-IL1R cell signaling pathway by the bispecific molecule that specifically binds IL-1R and IL-1β, wherein 4A is the HEK-Blue-IL1R cell signaling pathway induced by recombinant human IL-1β, 4B shows the inhibition of IL-1β-induced HEK-Blue-IL1R cell signaling pathway by aIL-1β-1, IL-1RA alone or in combination, and 4C-4F shows the inhibition of IL-1β-induced HEK-Blue-IL1R cell signaling pathway by the bispecific molecule (fusion of IL-1RA with a IL-1β-1).

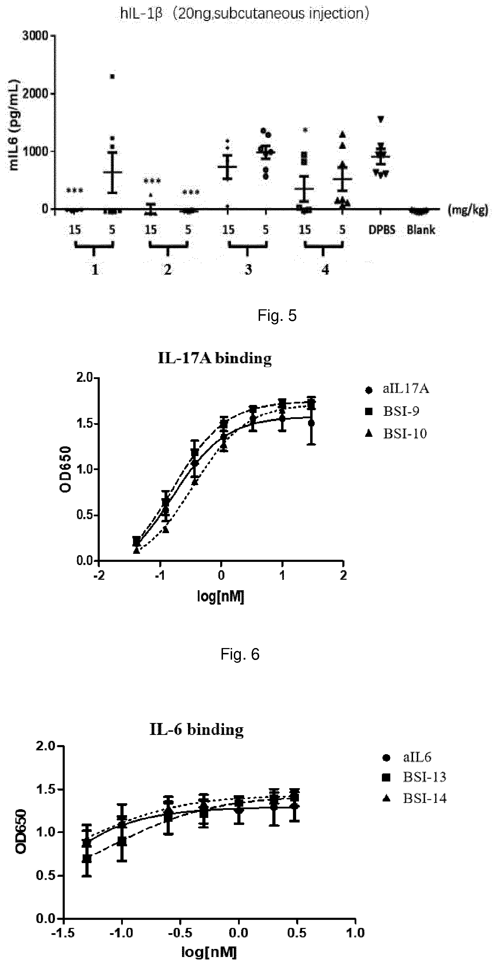

Fig. 5 shows the efficacy of the bispecific molecule that specifically neutralizing human IL-1β in vivo, wherein "Blank" is the group where mice were not treated with any drugs or human IL-1β; "DPBS " means that mice were injected with human IL-1β injection 24 hours after initial DPBS injection.; "1" means mice receiving aIL-1β-1 treatment, "2" means mice receiving bispecific molecule (IL-1RA and aIL-1β-1 fusion) treatment, "3" means mice receiving an isotype control antibody treatment, and "4" means mice receiving treatment of antibody where IL-1RA was infused with isotype antibody. Among them, "*": p<0.05 compared with the DPBS group; "**": p<0.005 compared with the DPBS group; and "***": p<0.0005 compared with the DPBS group.
Fig. 6 is the ELISA results of the bispecific molecule that specifically binds to IL-17.
Fig. 7 is the ELISA results of the bispecific molecule that specifically binds to IL-6.
Fig. 8 shows amino acid sequence alignment of heavy chain variable regions of three anti-IL-1R antibodies. Complementarity determining regions (CDRs) are underlined.
Fig. 9 shows amino acid sequence alignment of light chain variable regions of three anti-IL-1R antibodies. Complementarity determining regions (CDRs) are underlined.
Fig. 10 shows variable regions of a heavy chain and a light chain of aIL-1β-1 antibody, and complementarity determining regions (CDRs) are underlined.

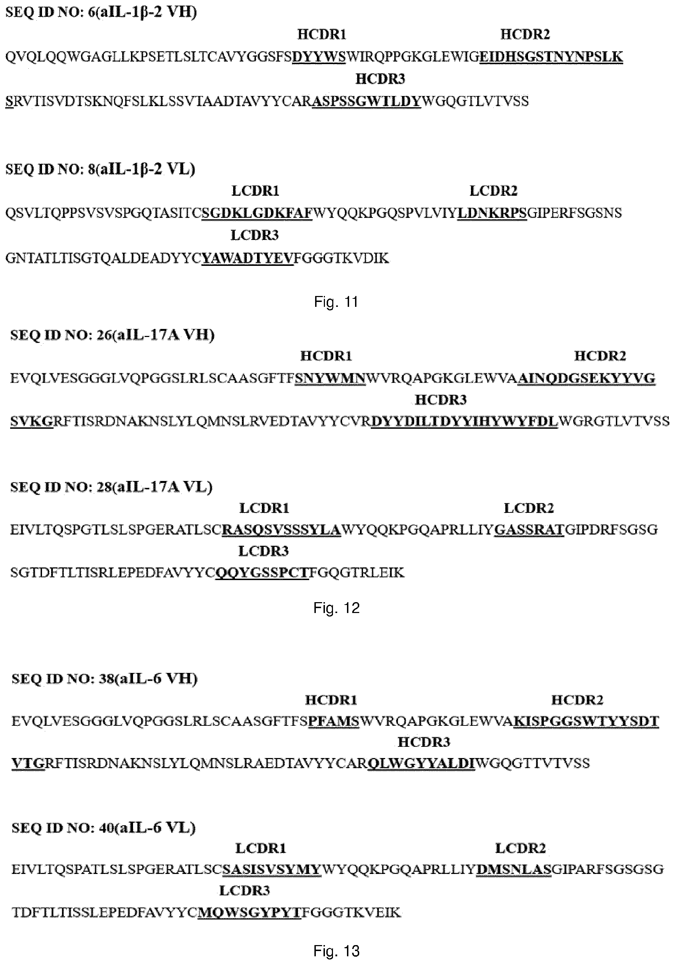

Fig. 11 shows variable regions of a heavy chain and a light chain of aIL-1β-2 antibody, and complementarity determining regions (CDRs) are underlined.
Fig. 12 shows variable regions of a heavy chain and a light chain of aIL-17A antibody, and complementarity determining regions (CDRs) are underlined.
Fig. 13 shows variable regions of a heavy chain and a light chain of aIL-16 antibody, and complementarity determining regions (CDRs) are underlined.

### Detailed Description of the Embodiments

The present invention is described in detail here by references using the following definitions and embodiments. The contents of all patents and disclosed documents mentioned in this article, including all sequences disclosed in these patents and disclosures, are expressly incorporated herein by reference.

### Bispecific molecule

The "bispecific molecule" of the present invention is a binding molecule with two binding specificities. It may be a non-immunoglobulin polypeptide with antigen binding specificity or an antibody with antigen binding specificity.

### Antibody

The "antibody" of the present invention refers to an immunoglobulin molecule, a fragment of the immunoglobulin molecule, or a derivative of any one of them, which has the ability to specifically bind an antigen under typical physiological conditions to induce, promote, enhance and/or regulate the physiological response associated with the binding of the antibody to the antigen. Unless otherwise specified or clearly contradictory in the context, the "antibody" of the present invention includes fragments of antibodies, which are antigen-binding fragments, namely, retains the ability to specifically bind the antigen.

### Molecule that binds IL-1R

The "molecule that specifically binds IL-1R" in the present invention refers to a non-immunoglobulin polypeptide or antibody that may compete with IL-1α and/or IL-1β to bind IL-1R and inhibit the biological activity of IL-1R. Other single domain antibodies or heavy chain antibodies that may compete with IL-1α and/or IL-1β to bind IL-1R and inhibit the biological activity of IL-1R are also included in the present invention.

### Inflammatory factors

As used in the present invention, the "inflammatory factor" is a general term for cytokines involved in the occurrence and development of inflammation. The "free inflammatory factor" refers to an inflammatory factor that exists in a body in the form of solution and is not expressed on the cell surface. The common free inflammatory factor includes but is not limited to the following examples: IL-1 superfamily (IL-1α, IL-1β, IL-18, IL-33, IL-36α, IL-36β, IL-36y), IL-4, IL-13, IL-17A, IL-17E, IL-6, IL-12, IL-23, TNF superfamily (TNFα, TNPβ, TNFγ, OX40L (TNFSF4), CD40L (CD154), FasL (CD178, CD95L), CD27L (CD70), CD30L (CD153), 4-1 BBL, CD253 (APO-2L) ), CD254, APO-3L (DR3L), CD256 (TALL-2), CD257 (BlyS), LIGHT (CD258), TL1 (TNFSF18, AITRL), ED1-A1), BAFF, IFN or GM-CSF and the like. The inflammatory factor receptor refers to a molecule that may bind to the inflammatory factor and is expressed on the cell surface.

### CDR

The term "CDR" refers to a complementarity determining region within an immunoglobulin variable region sequence. For each heavy and light chain variable region, there are three CDRs, which are named as CDR1, CDR2, and CDR3. A term "CDR set" refers to a set of three CDRs that appear in a single variable region capable of binding antigen. Exact boundaries of these CDRs are defined differently according to different systems. A system described by Kabat (Kabat et al. (1987) and (1991)) not only provides a clear residue numbering system that may be applied to any variable region of an antibody or binding protein, but also provides a precise definition of residue boundaries of the three CDRs in each heavy chain or light chain sequence. These CDRs may be referred to as Kabat CDRs. Chothia and colleagues (Chothia and Lesk (1987) J. Mol. Biol. 196: 901-917; Chothia et al. (1989) Nature 342: 877-883) found that certain sub-parts in Kabat CDR adopt almost the same peptide framework although with great diversity at the amino acid sequence level. These sub-parts are named L1, L2, and L3 or H1, H2, and H3, where "L" and "H" refer to the light chain and heavy chain regions, respectively. These regions may be referred to as Chothia CDRs, which have borders that overlap with Kabat CDRs. Other boundaries defining CDRs that overlap with Kabat CDRs are described by Padlan (1995) FASEB J. 9: 133-139 and MacCallum (1996) J. Mol. Biol. 262(5): 732-45). There are other CDR boundary definitions that may not strictly follow one of the systems in this article, but may still overlap with Kabat CDRs, although they may be shortened or lengthen by considering the fact that a specific residue or a residue group or even the entire CDRs do not significantly affect the prediction of antigen binding or experimental discovery. The methods used herein may utilize CDRs defined according to any of these systems, although some embodiments use Kabat or Chothia defined CDRs (CN105324396 A).

A term "homology" or "identity" refers to sequence identity of primary unit between two polymer molecules (for example, between two nucleic acid molecules (for example, between two DNA molecules or two RNA molecules) or between two polypeptide molecules). While the position of the primary unit in these two molecules is occupied by the same monomeric subunit, for example, if a certain position in each molecule of two DNA molecules is occupied by adenine, they are homologous or the same at that position. The identity between two sequences varies directly with the number of matching positions or homologous positions. For example, if half of the positions in two sequences (for example, five positions in a polymer of ten subunits in length) are homologous, the two sequences are 50% homologous (also called 50% homology or 50% identity); if 90% of the positions (for example, 9 positions in 10 positions) are matched or homologous, these two sequences are 90% homologous (also called 90% homology or 90% identity).

### Inflammatory disease

The "inflammatory disease" as used in the present application refers to a disease related to an inflammatory response. Examples of the inflammatory disease include arthritis such as RA, psoriatic arthritis, ankylosing spondylitis, juvenile idiopathic arthritis and other inflammatory diseases of the joints, inflammatory bowel diseases such as ulcerative colitis, Crohn's disease, Barrett's syndrome, ileitis, enteritis, gluten-sensitive enteropathy, inflammatory diseases of the respiratory system, such as asthma, adult respiratory distress syndrome, allergic rhinitis, silicosis, chronic respiratory obstructive diseases, hypersensitivity lung diseases, bronchiectasis; inflammatory diseases of the inflammatory skin, including psoriasis, scleroderma, and inflammatory skin diseases such as eczema, atopic dermatitis, urticaria and pruritus; diseases involving central and peripheral nervous system inflammation include multiple sclerosis, idiopathic demyelinating polyneuropathy, Guillain-Barre syndrome, chronic inflammatory demyelinating polyneuropathy and neurodegenerative diseases such as Alzheimer's disease; systemic lupus erythematosus, immune-mediated kidney disease, such as glomerulonephritis and spondyloarthropathy; diseases with undesirable chronic inflammatory components, such as systemic sclerosis, characteristic inflammatory myopathy, Sjogren's syndrome, vasculitis, sarcomatoid disease, thyroiditis, gout, otitis, conjunctivitis, sinusitis, sarcomatoid disease, Behcet's syndrome, hepatobiliary diseases such as hepatitis, primary biliary cirrhosis, granulomatous hepatitis and sclerosing cholangitis; inflammation and ischemic damage of the cardiovascular system such as ischemic heart disease, stroke and atherosclerosis; and graft rejection, including allograft rejection and graft-versus-host disease. Various other inflammatory diseases include tuberculosis and chronic cholecystitis. For example, other chronic inflammatory diseases are also described in Harrison's Principles of Internal Medicine (12th Edition, Wilson, et al., eds., McGrawill, Inc.).

### Pharmaceutical composition

The pharmaceutical composition as described herein is prepared by mixing the bispecific antibody of the present invention with the desired purity and one or more optional pharmaceutically acceptable carriers, which is in the form of a lyophilized formulation or an aqueous solution. The pharmaceutically acceptable carrier is generally non-toxic to the recipient at dose and concentration used.

The present invention may be administered as an active ingredient alone, or in combination with, for example, an adjuvant or with other drugs such as immunosuppressive or immunomodulatory agents or other anti-inflammatory agents, for example, for the treatment of an inflammatory disease and/or an immune disease.

### Examples

It should be noted that, in the case of no conflict, the embodiments in the present application are only illustrative, and are not intended to limit the present invention in any manners.

### Example 1: Construction, expression, purification and functional detection of bispecific molecules that specifically binds IL-1R and IL-1β

### Example 1.1: Cloning of bispecific molecules that specifically binds IL-1R and IL-1β

Gene fragments encoding the heavy chain (HC) and the light chain (LC) of the aIL-1R-1, alL-1R-2, aIL-1R-3, aIL-1β-1, aIL-1β-2 antibodies and IL-1RA were synthesized, respectively. IL-1RA was fused to the N-terminal or C-terminal of heavy chain or light chain of the aIL-1β antibody (aIL-1β-1 and aIL-1β-2) by a linker peptide. VH and VL of the aIL-1R antibody were fused to the N-terminal of the heavy and light chains of the aIL-1β antibody by the linker peptide, respectively, or VH and VL of the aIL-1β antibody were fused to the N-terminal of the heavy chain and N-terminal of the light chain of the aIL-1R by the linker peptide, respectively. All the sequences were verified by sequencing.

**Table 1: Bispecific molecule nucleic acid and sequence**

| Construct | Composition | Nucleotide sequence Seq ID No: | Amino acid sequence Seq ID No: |
|---|---|---|---|
| aIL1β-1 | chain-1 | 1 | 2 |
| | chain-2 | 3 | 4 |
| aIL1 β-2 | chain-1 | 5 | 6 |
| | chain-2 | 7 | 8 |
| BSI-1 | chain-1 | 9 | 10 |
| | chain-2 | 3 | 4 |
| BSI-2 | chain-1 | 1 | 2 |
| | chain-2 | 11 | 12 |
| BSI-3 | chain-1 | 13 | 14 |
| | chain-2 | 3 | 4 |
| BSI-4 | chain-1 | 1 | 2 |
| | chain-2 | 15 | 16 |
| BSI-5 | chain-1 | 17 | 18 |
| | chain-2 | 3 | 4 |
| BSI-6 | chain-1 | 1 | 2 |
| | chain-2 | 19 | 20 |
| BSI-7 | chain-1 | 5 | 6 |
| | chain-2 | 21 | 22 |
| BSI-8 | chain-1 | 5 | 6 |
| | chain-2 | 23 | 24 |
| aIL-17A | chain-1 | 25 | 26 |
| | chain-2 | 27 | 28 |
| BSI-9 | chain-1 | 33 | 34 |
| | chain-2 | 27 | 28 |
| BSI-10 | chain-1 | 25 | 26 |
| | chain-2 | 35 | 36 |
| BSI-11 | chain-1 | 29 | 30 |
| | chain-2 | 27 | 28 |
| BSI-12 | chain-1 | 25 | 26 |
| | chain-2 | 31 | 32 |
| BSI-13 | chain-1 | 45 | 46 |
| | chain-2 | 39 | 40 |
| BSI-14 | chain-1 | 37 | 38 |
| | chain-2 | 47 | 48 |
| BSI-15 | chain-1 | 37 | 38 |
| | chain-2 | 43 | 44 |
| BSI-16 | chain-1 | 41 | 42 |
| | chain-2 | 39 | 40 |
| aIL-6 | chain-1 | 37 | 38 |
| | chain-2 | 39 | 40 |
| IL-IRA | / | 49 | 50 |
| aIL-IR-1 | chain-1 | 51 | 52 |
| | chain-2 | 53 | 54 |
| aIL-IR-2 | chain-1 | 55 | 56 |
| | chain-2 | 53 | 54 |
| aIL-IR-3 | chain-1 | 57 | 58 |
| | chain-2 | 59 | 60 |
| BSI-17 | chain-1 | 61 | 62 |
| | chain-2 | 3 | 4 |
| BSI-18 | chain-1 | 63 | 64 |
| | chain-2 | 3 | 4 |
| BSI-19 | chain-1 | 65 | 66 |
| | chain-2 | 7 | 8 |
| BSI-20 | chain-1 | 5 | 6 |
| | chain-2 | 67 | 68 |
| BSI-21 | chain-1 | 69 | 70 |
| | chain-2 | 3 | 4 |
| BSI-22 | chain-1 | 71 | 72 |
| | chain-2 | 3 | 4 |
| BSI-23 | chain-1 | 73 | 74 |
| | chain-2 | 3 | 4 |
| BSI-24 | chain-1 | 75 | 76 |
| | chain-2 | 3 | 4 |

### Example 1.2: Expression and purification of bispecific molecules that specifically binds IL-1R and IL-1β

The heavy chain and light chain of the expression vector constructed in Example 1.1 were transiently transfected into Free Style ^{™} HEK293 cells (Thermo Fisher) (the amount of a heavy chain plasmid and a light chain plasmid during transfection was at a molar ratio of 1:1): 28 ml of Free Style ^{™} HEK293 (a total of 3×10⁷ cells) were seeded in a 125 ml cell culture flask, plasmids were diluted with 1 ml of Opti-MEM (Invitrogen), and then added to 1 ml of Opti-MEM containing 60 µl 293Fectin (Invitrogen). After standing at room temperature for 30 min, the plasmid-293Fectin mixture was added to the cell culture medium, then incubated at 125 rpm, 37°C, 5% CO₂. Cell culture supernatant was collected at 48 h and 96 h after transfection, respectively, and purified by Protein A Resin (Thermo Fisher Scientific). The purified proteins were analyzed by SDS-PAGE. Results are shown in Fig. 1A, indicating that the bispecific molecule was successfully expressed.

### Example 1.3: Mass spectrometry of bispecific molecules that specifically binds IL-1R and IL-1β

The purified proteins in Example 1.2 were incubated with PNGase F (NEB) at 37°C for 8 hours, and treated with 10 mM dithiothreitol (DTT), then the sample were injected into 300SB-C8, 2.1 x 50 mm column of HPLC-Q-TOF-MS (Agilent, USA) for mass spectrometry analysis. As shown in Table 2, the molecular weight of antibody fusion protein in different fusion forms detected by the mass spectrometry is consistent with the theoretical value.

**Table 2: Theoretical molecular weight of bispecific molecule and MS detection molecular weight**

| Bispecific molecule | Chain-1 molecular weight (D) | | Chain-2 molecular weight (D) | |
|---|---|---|---|---|
| | Theoretical value | Detection value | Theoretical value | Detection value |
| BSI-1 | 67919.69 | 67916.04 | 23357.97 | 23354.64 |
| BSI-2 | 48912.7 | 48889.73 | 42365.39 | 42361.82 |
| BSI-3 | 69656.64 | 69651.17 | 23357.97 | 23355.01 |
| BSI-4 | 48912.7 | 48888.88 | 44102.44 | 44098.27 |
| BSI-5 | 66910.52 | 66887.14 | 23357.97 | 23355.01 |
| BSI-6 | 48912.7 | 48889.19 | 41228.15 | 41223.56 |
| BSI-7 | 48799.94 | 48776.2 | 42131.15 | 42126.9 |
| BSI-8 | 48799.94 | 48776.1 | 43868.1 | 43863.5 |

### Example 1.4: Analysis of in vitro activity of bispecific molecules that specifically binds IL-1R and IL-1β

Recombinant human IL-1β (Thermo Fisher Scientific), recombinant mouse IL-1β (Sino Bio), recombinant mouse IL-1R (Sino Bio), or recombinant human IL-1R (100ng/well) (DPBS buffer, pH7.4) was coated in a 96-well plate, and incubated overnight at 4°C. After blocked by PBST (0.5% Tween-20 in PBS) containing 2% skimmed milk powder for 1 hour at room temperature, andbeing washed with PBST for 3 times, the gradient diluted bispecific molecules (0.006nM-100nM) were added and incubated at 37°C for 1 h. After being washed with PBST for 3 times, anti-human Fc-HRP (KPL; 1 :2500) secondary antibody diluted by 2% milk/PBST was added and incubated at room temperature for 1 h, and then washed by PBST for 5 times. QuantaBlu fluorescent peroxide enzyme substrate (Life technologies, Cat.15169) was used for color development, and reading was performed at 325 nm and 420 nm, or TMB color reagent (Bio Legend) was used for color development, and the reading was performed at 650 nm. Data was analyzed by nonlinear regression using specific binding model in Prizm Graphpad software.

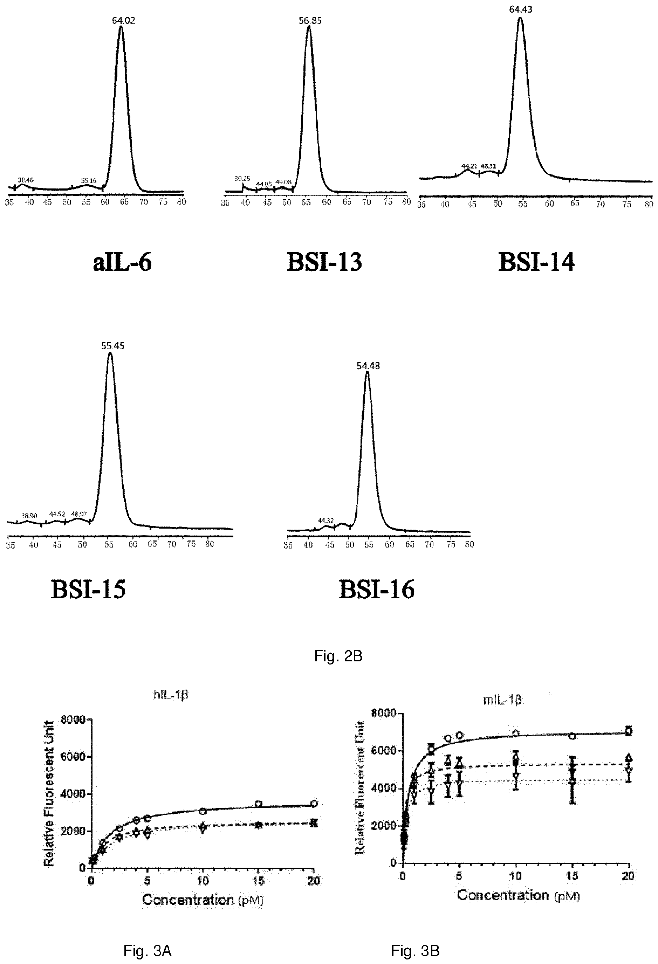

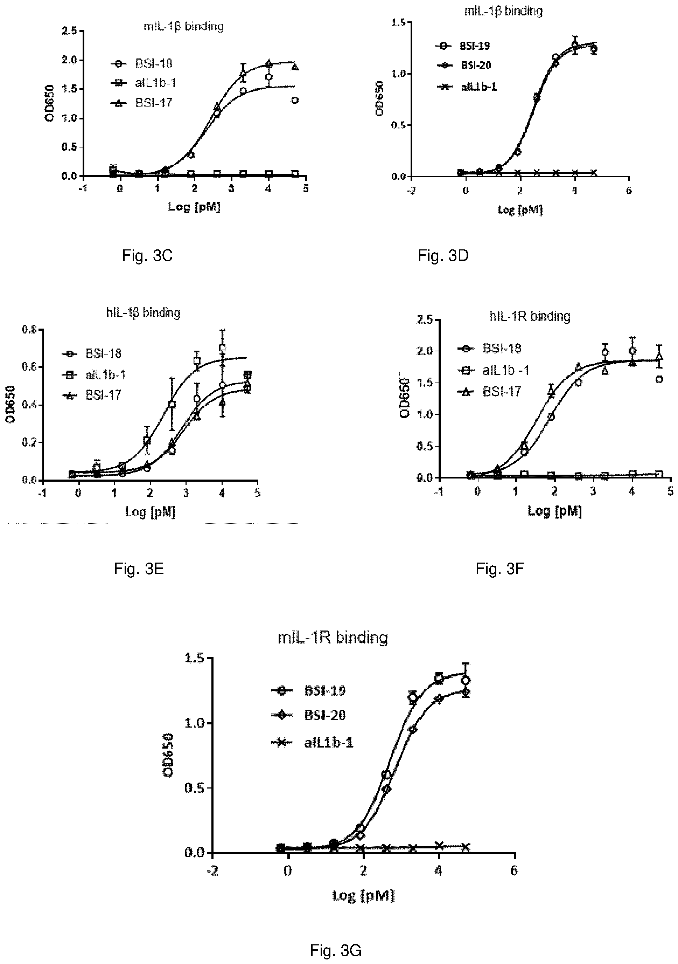

As shown in Fig. 3. The binding ability of the bispecific molecule to human IL-1β or murine IL-1β is similar to that of the aIL-1β-2 antibody, indicating that the fusion of IL-1RA has little effect on the binding of aIL-1β antibody with IL-1β. The binding ability of the bispecific molecule to human IL-1β is approximately 5-7 times greater than that to mouse IL-1β.

### Example 1.5: Analysis of Inhibition of IL-1β-induced HEK-Blue-IL1R cell activation by bispecific molecules

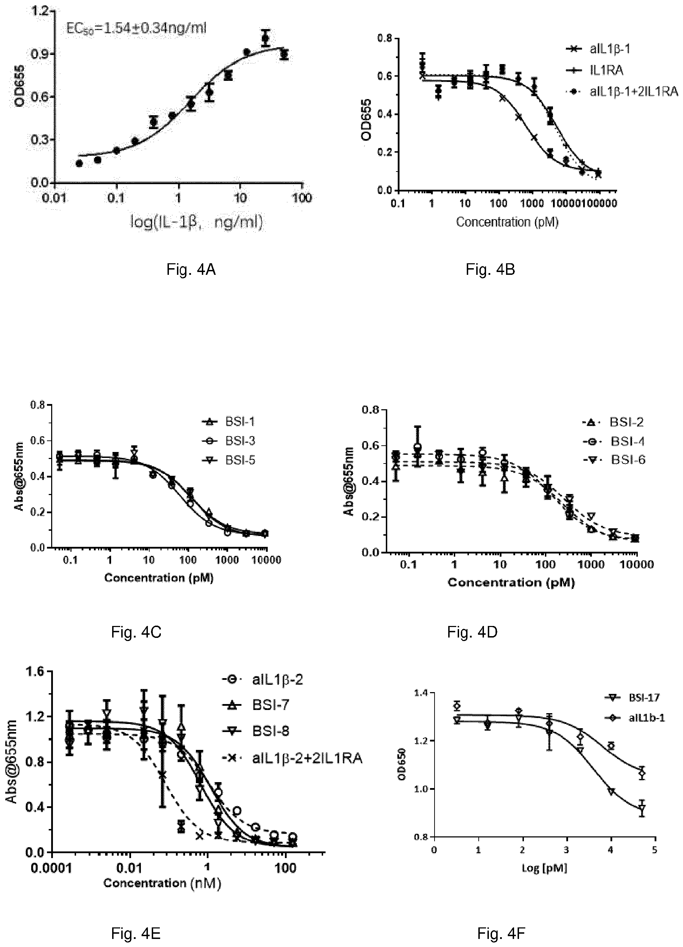

5x10⁴ HEK-Blue-IL1R cells (InvivoGene) were seeded on a 96-well plate, and 100 ul of IL-1β (R&D systems) (diluted with 5% FBS/DMEM medium) in gradient dilutions (0.024-50ng/ml) was added into per well and cultured overnight. Cell supernatant was collected and mixed with QUANTI-Blue (InvivoGene) at a ratio of 1:9. After incubating at 37°C for 4 hours, the absorbance at 655 nm was read at Spectramax fluorescence plate reader. Data was analyzed with log[agonist] vs response (3 parameters) model by nonlinear regression in Prizm Graphpad software. As shown in Fig. 4A, IL-1β could stimulate the HEK-Blue-IL1R cell pathway with EC₅₀ 1.54±0.35ng/ml. Fig. 4B-4F shows that after IL-1RA was fused to aIL-1β antibody (aIL-1β-1 or aIL-1β-2), its ability to inhibit IL-1β-induced HEK-Blue-IL1R cell signaling pathway is stronger than IL-1RA, aIL-1β antibody alone or IL-1RA+aIL-1β in combination, indicating that IL-1RA and aIL-1β in the bispecific molecule have a synergistic inhibitory effect on IL-1R.

### Example 1.6: In vivo neutralization efficacy of bispecific molecules that specifically binds IL-1R and IL-1β.

DPBS or bispecific molecule (two doses: 5mg/kg, 15mg/kg) was injected intraperitoneally into female C57BL/6 mice (6-7 weeks). After 24 hours, each mouse was injected with 20 ng of recombinant human IL-1β (PHC0815, Thermo Fisher Scientific). After 2 hours, blood was taken from the posterior eye socket, and the level of mIL-6 in mouse plasma was detected by Mouse IL-6 ELISA MAX^{™} Deluxe (Cat.431306, BioLegend) according to instructions provided by a manufacturer. Data was analyzed with Graphpad Prizm software. For statistical analysis, unpaired, and two-tailed t test was performed.

As shown in Fig. 5, IL-6 levels in the DPBS group are significantly increased when comparing with the Blank group. Bispecific molecules with fusion of IL-1 RA and aIL-1β-1 at 15mg/ml and 5mg/ml significantly inhibit IL-1β-induced up-regulation of mouse IL-6, while the protein (fusion of IL-1RA with the isotype fusion antibody) could only inhibit IL-1β-induced up-regulation of mouse IL-6 to a certain extent at 15mg/ml.

### Example 2: Construction, expression, purification and functional detection of bispecific molecules that specifically binds IL-1R and IL-17

### Example 2.1: Cloning of bispecific molecules that specifically binds IL-1R and IL-17

Gene fragments encoding the heavy chain and light chain of aIL-17 antibody were synthesized, respectively, and IL-1RA was fused to N-terminal or C-terminal the heavy chain or light chain of the aIL-17 antibody through the linker peptide L1, L2 or L3 using standard molecular biology technology. VH and VL of the aIL-1R antibody was fused to the N-terminal of the heavy chain and light chain of the aIL-17 antibody through the linker peptides L4 and L5, respectively, or VH and VL of the aIL-17 antibody was fused to the N-terminal of the heavy chain and N-terminal of the light chain of the alL-1R antibody, respectively. All the sequences were verified by sequencing.

### Example 2.2: Expression, purification and analysis of bispecific molecules that specifically binds IL-1R and IL-17

The heavy chain and light chain of the expression vector constructed in Example 2.1 were transiently transfected into Free Style ^{™} HEK293 cells (Thermo Fisher), and the amount of the heavy chain plasmid and the light chain plasmid during transfection was at a molar ratio of 1:1: 28 ml of Free Style ^{™} HEK 293 (a total of 3×10⁷ cells) were seeded in a 125 ml cell culture flask, the plasmid was diluted with 1 ml of Opti-MEM (Invitrogen) and then added to 1 ml of Opti-MEM containing 60µl 293Fectin (Invitrogen). After standing at room temperature for 30 min, the plasmid-293Fectin mixture was added to the cell culture medium, and then incubated at 125 rpm, 37°C, 5% CO₂. Cell culture supernatant was collected at 48 h and 96 h after transfection, respectively, and purified by Protein A Resin (Thermo Fisher Scientific). The purified proteins were analyzed by SDS-PAGE. As shown in Fig. 1B, the size of each lane is consistent with the expectation indicating that the bispecific molecule was successfully expressed.

In order to further investigate the physical properties of the bispecific molecule in solution, GE's AKTA was used for size exclusion chromatography. The column used was HiLoad 16/600 Superdex 200, and the solution used was DPBS buffer (0.010M phosphate buffer, 0.0027M KCI, 0.14M NaCl, pH7.4) with a flow rate at 0.4 ml/min.

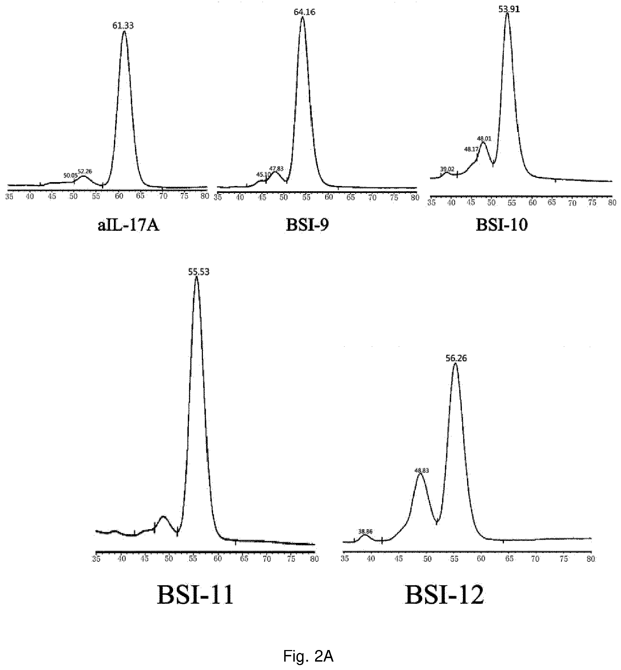

Results from Fig. 2A show that the purified bispecific molecule mainly exists in the form of monomer and has high purity without apparent visible aggregation.

### Example 2.3: In vitro activity of bispecific molecule that specifically binds IL-1R and IL-17

Recombinant human IL-17 (SinoBiological) (100ng/well) (PBS buffer, pH7.4) was coated in a 96-well plate, and incubated overnight at 4°C. After blocked with PBST containing 2% skimmed milk powder (0.5% Tween-20 in PBS) at room temperature for 1 hour and followed by being washed for 3 times with PBST, the gradient diluted bispecific molecules (0.04nM-30nM) were added and incubated at 37°C for 1 h. After being washed for 3 times with PBST, anti-human Fc-HRP (KPL, 1 :2500) secondary antibody diluted by 2% milk/PBST was added and incubated at room temperature for 1 h. After being washed for 5 times with PBST, TMB (BioLegend) was used for color development, and readings were performed in Thermal VARIOSKAN FLASH plate reader at 650 nm. Data was analyzed with a log[agonist] vs response (3 parameters) model by "non-linear regression" in Graphpad Prizm software.

The ELISA results in Fig. 6 show that the fusion of IL-1RA to the heavy or light chain of aIL-17A antibody does not affect the affinity of the aIL-17A antibody to IL-17, and the affinity of bispecific molecule to IL-17 is similar to that of aIL-17A antibody.

### Example 3: Construction, expression, purification and functional detection of bispecific molecules that specifically binds IL-1R and IL-6.

### Example 3.1: Cloning of bispecific molecules that specifically binds IL-1R and IL-6

Gene fragments encoding a heavy chain and a light chain of the aIL-6 antibody were synthesized, and IL-1RA was fused to N-terminal or C-terminal of the heavy chain or the light chain of the aIL-6 antibody through the linker peptide L1, L2 or L3 using standard molecular biology technology. VH and VL of the aIL-1R antibody was fused to the N-terminal of the heavy chain and light chain of the aIL-6 antibody through the linker peptides L4 and L5, respectively, or VH and VL of the aIL-6 antibody was fused to the N-terminal of the heavy chain and N-terminal of the light chain of the aIL-1R antibody, respectively. All the sequences were verified by sequencing.

### Example 3.2: Expression, purification and analysis of bispecific molecules that specifically binds IL-1R and IL-6

The heavy chain and light chain of the expression vector constructed in Example 3.1 were transiently transfected into Free Style ^{™} HEK293 cells (Thermo Fisher), and the amount of the heavy chain plasmid and the light chain plasmid during transfection was at a molar ratio of 1:1: 28 ml of Free Style ^{™} HEK 293 (a total of 3×10⁷ cells) were seeded in a 125 ml cell culture flask, the plasmid was diluted with 1 ml of Opti-MEM (Invitrogen) and then added to 1 ml of Opti-MEM containing 60µl 293Fectin (Invitrogen). After standing at room temperature for 30 min, the plasmid-293Fectin mixture was added to the cell culture medium, and incubated at 125 rpm, 37°C, 5% CO₂. Cell culture supernatant was collected at 48 h and 96 h after transfection, respectively, and purified by Protein A Resin (Thermo Fisher Scientific). The purified proteins were analyzed by SDS-PAGE. As shown in Fig. 1C, the size of each lane is consistent with the expectation, indicating that the bispecific molecule was successfully expressed.

In order to further investigate the physical properties of the bispecific molecule in solution, GE' AKTA was used for gel exclusion chromatography. The column used was HiLoad 16/600 Superdex 200, and the solution used was DPBS buffer (0.010M phosphate buffer, 0.0027M KCI, 0.14M NaCl, pH7.4) with a flow rate at 0.4 ml/min.

The size exclusion chromatography results of Fig. 2B show that the bispecific molecule mainly exists in the form of a monomer and has high purity without apparent visible aggregation.

### Example 3.3: In vitro activities of bispecific molecule that specifically binds IL-1R and IL-6

Recombinant human IL-6 (SinoBiological) (100ng/well) (PBS buffer, pH7.4) was coated in a 96-well plate, and incubated overnight at 4°C. After blocked with PBST containing 2% skimmed milk powder (0.5% Tween-20in PBS) at room temperature for 1 hour, followed by being washed for 3 times with PBST, the bispecific molecules (0.04nM-30nM) in gradient dilutions were added and incubated at 37°C for 1 h. After being washed for 3 times with PBST, anti-human Fc-HRP (KPL, 1 :2500) secondary antibody diluted by 2% milk/PBST was added and incubated at room temperature for 1 h, followed by being washed for 5 times with PBST. TMB (BioLegend) was used for color development, and readings was performed in Thermal VARIOSKAN FLASH plate reader at 650 nm. Data was analyzed with a log[agonist]vs response (3 parameters) model in "non-linear regression" by Graphpad Prizm software.

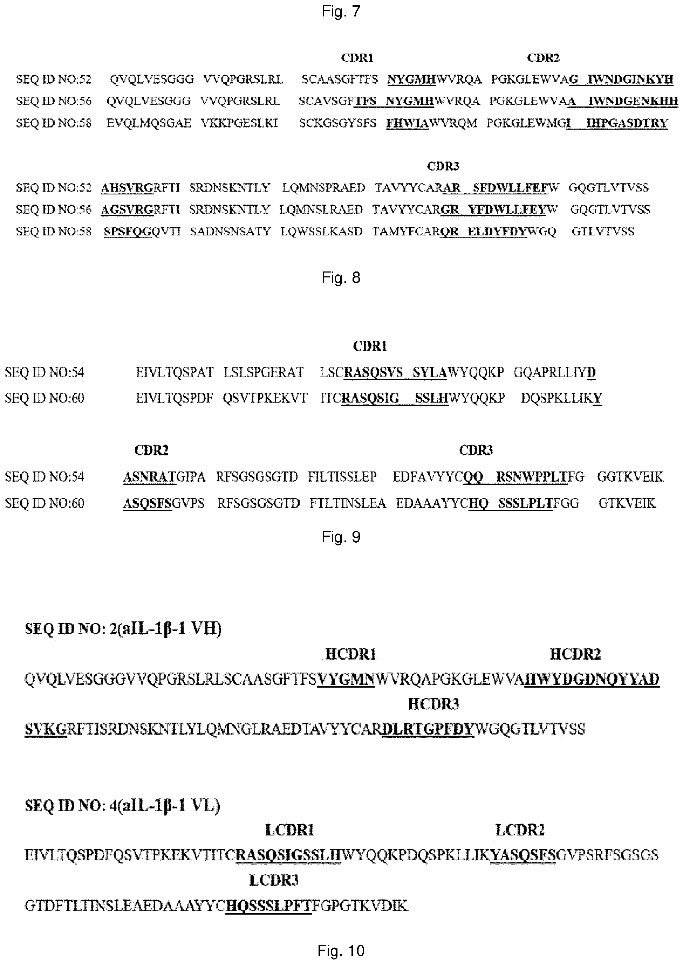

The ELISA results in Fig. 7 show that the fusion of IL-1RA to the heavy or light chain of the alL-6 antibody does not affect the affinity of the aIL-6 antibody to IL-6, and the affinity of the bispecific molecule to IL-6 is similar to that of aIL-6 antibody.

## Claims

1. A bispecific molecule, comprising:
a) a molecule that specifically binds IL-1R; and
b) an antibody that targets a free inflammatory factor;
wherein, the molecule that specifically binds IL-1R and the antibody that targets the inflammatory factor are connected by a linker peptide.

2. The bispecific molecule according to claim 1, wherein the molecule that specifically binds IL-1R is a non-immunoglobulin polypeptide.

3. The bispecific molecule according to claim 2, wherein the non-immunoglobulin polypeptide that specifically binds IL-1R is IL-1RA.

4. The bispecific molecule according to claim 2, wherein the non-immunoglobulin polypeptide that specifically binds IL-1R comprises an amino acid sequence having 85%-99% of identity with SEQ ID NO: 50, or consists of such an amino acid sequence.

5. The bispecific molecule according to claim 1, wherein the molecule that specifically binds IL-1R is an anti-IL-1R antibody.

6. The bispecific molecule according to claim 5, wherein the anti-IL-1R antibody is a single domain antibody, a chimeric antibody, a humanized antibody, a human antibody, or a modified fragment of the above antibodies.

7. The bispecific molecule according to claim 5, wherein the anti-IL-1R antibody comprises CDR groups: HCDR1, HCDR2, HCDR3, LCDR1, LCDR2 and LCDR3, wherein,
a) HCDR1, HCDR2 and HCDR3 have the HCDR1, HCDR2 and HCDR3 sequences as contained in SEQ ID NO: 52 respectively; LCDR1, LCDR2 and LCDR3 have the LCDR1, LCDR2 and LCDR3 sequences as contained in SEQ ID NO: 54respectively;
b) HCDR1, HCDR2 and HCDR3 have the HCDR1, HCDR2 and HCDR3 sequence as contained in SEQ ID NO: 56 respectively; LCDR1, LCDR2 and LCDR3 have the LCDR1, LCDR2 and LCDR3 sequences as contained in SEQ ID NO: 54 respectively; or
c) HCDR1, HCDR2 and HCDR3 have the HCDR1, HCDR2 and HCDR3 sequences contained in SEQ ID NO: 58 respectively; LCDR1, LCDR2 and LCDR3 have the LCDR1, LCDR2 and LCDR3 sequences as contained in SEQ ID NO: 60.

8. The bispecific molecule according to claim 5, wherein the anti-IL-1R antibody has a heavy chain and a light chain of the following:
a) a heavy chain shown in SEQ ID NO: 52 and a light chain shown in SEQ ID NO: 54;
b) a heavy chain shown in SEQ ID NO: 56 and a light chain shown in SEQ ID NO: 54; or
c) a heavy chain shown in SEQ ID NO: 58 and a light chain shown in SEQ ID NO: 60.

9. The bispecific molecule according to any one of claims 1-6, wherein the free inflammatory factor is selected from one of the group consisting of:
IL-1 superfamily (IL-Ia, IL-1β, IL-18, IL-33, IL-36a, IL-36β, IL-36y), IL-4, IL-13, IL-17A , IL-17E, IL-6, IL-12, IL-23, TNF superfamily (TNFa, TNFβ, TNFγ, OX40L (TNFSF4), CD40L (CD154), FasL (CD178, CD95L), CD27L (CD70), CD30L(CD153), 4-1BBL, CD253 (APO-2L), CD254, APO-3L(DR3L), CD256(TALL-2), CD257(B1yS), LIGHT(CD258), TL1 (TNFSF18, AITRL), ED1-A1), BAFF, IFN or GM-CSF.

10. The bispecific molecule according to claim 9, wherein the free inflammatory factor is IL-1β.

11. The bispecific molecule according to claim 10, wherein the antibody that targets the free inflammatory factor IL-1β has the HCDR1, HCDR2 and HCDR3 sequences contained in a heavy chain amino acid sequence shown in SEQ ID NO: 2, and the LCDR1, LCDR2 and LCDR3 sequences contained in a light chain amino acid sequence shown in SEQ ID NO: 4; or the HCDRI, HCDR2 and HCDR3 sequences contained in a heavy chain amino acid sequence shown in SEQ ID NO: 6 , and the LCDR1, LCDR2 and LCDR3 sequences contained in a light chain amino acid sequence shown in SEQ ID NO: 8.

12. The bispecific molecule according to claim 9, wherein the free inflammatory factor is IL-17.

13. The bispecific molecule according to claim 12, wherein the antibody that targets the free inflammatory factor IL-17 has the HCDR1, HCDR2 and HCDR3 sequences contained in a heavy chain amino acid sequence shown in SEQ ID NO: 26, and the LCDR1, LCDR2 and LCDR3 sequences contained in a light chain amino acid sequence shown in SEQ ID NO: 28.

14. The bispecific molecule according to claim 9, wherein the free inflammatory factor is IL-6.

15. The bispecific molecule according to claim 14, wherein the antibody that targets the free inflammatory factor IL-6 has the HCDR1, HCDR2 and HCDR3 sequences contained in a heavy chain amino acid sequence shown in SEQ ID NO: 38, and the LCDR1, LCDR2 and LCDR3 sequences contained in a light chain amino acid sequence shown in SEQ ID NO:40.

16. The bispecific molecule according to any one of claims 1-15, wherein the antibody that targets the free inflammatory factor is a chimeric antibody, a humanized antibody, a human antibody, or a modified fragment of the above antibodies.

17. The bispecific molecule according to claim 2, wherein the bispecific molecule comprises an antibody that targets the free inflammatory factor IL-1β, and the bispecific molecule comprises a heavy chain and a light chain each having the amino acid sequences selected from any one of the group consisting of: SEQ ID NO: 10 and SEQ ID N0: 4; SEQ ID NO: 2 and SEQ ID NO: 12; SEQ ID NO: 14 and SEQ ID NO: 4; SEQ ID NO: 2 and SEQ ID NO: 16; SEQ ID NO: 18 and SEQ ID NO: 4; SEQ ID NO: 2 and SEQ ID NO: 20; SEQ ID NO: 6 and SEQ ID NO: 22; SEQ ID NO: 6 and SEQ ID NO: 24; SEQ ID NO: 62 and SEQ ID NO: 4; SEQ ID NO: 64 and SEQ ID NO: 4; SEQ ID NO: 66 and SEQ ID NO: 8; SEQ ID NO: 6 and SEQ ID NO: 68; SEQ ID NO: 70 and SEQ ID NO: 4; SEQ ID NO: 72 and SEQ ID NO: 4; SEQ ID NO: 74 and SEQ. NO: 4; SEQ ID NO: 76 and SEQ ID NO: 4.

18. The bispecific molecule according to claim 2, wherein the bispecific molecule comprises an antibody that targets the free inflammatory factor IL-17, and the bispecific molecule comprises a heavy chain and a light chain each having the amino acid sequences selected from any one of the group consisting of: SEQ ID NO: 34 and SEQ ID NO: 28; SEQ ID NO: 26 and SEQ ID NO: 36; SEQ ID NO: 30 and SEQ ID NO: 28; SEQ ID NO: 26 and SEQ ID NO: 32.

19. The bispecific molecule according to claim 2, wherein the bispecific molecule comprises an antibody that targets the free inflammatory factor IL-6, and the bispecific molecule comprises a heavy chain and a light chain each having the amino acid sequences selected from any one of the group consisting of: SEQ ID NO: 46 and SEQ ID NO: 40; SEQ ID NO: 38 and SEQ ID NO: 48; SEQ ID NO: 38 and SEQ ID NO: 44; SEQ ID NO: 42 and SEQ ID NO: 40.

20. A nucleic acid, encoding the bispecific molecule according to any one of claims 1-22, or a heavy chain or a light chain thereof.

21. An expression vector, comprising the nucleic acid according to claim 23.

22. A host cell, comprising the expression vector according to claim 24.

23. A pharmaceutical composition, comprising the bispecific molecule according to any one of claims 1-22 and a pharmaceutically acceptable carrier or preparation.

24. A method for treating an inflammatory disease and/or an autoimmune disease in a subject in need thereof, the method comprises administering a therapeutically effective amount of a composition to the subject, and the composition comprises the bispecific molecule in a pharmaceutically acceptable form according to any one of claims 1-19.
